# EUROPEAN PATENT APPLICATION

(11) **EP 0 780 126 A1**
(43) Date of publication of application: **25.06.1997**
(21) Application number: 95929212.9
(22) Date of filing: 23.08.1995
(51) Int. Cl.: A61K 31/52, A61K 9/06

(54) **OINTMENT**

(30) Priority: 06.09.1994 JP 236032/94
(71) Applicant: NIPPON KAYAKU KABUSHIKI KAISHA, Tokyo 102 (JP)
(72) Inventor: OHTSUKI, Kazuo, Tokyo 115 (JP); KOMURO, Chikara, Tokyo 144 (JP); AOUDA, Yukio, Tokyo 175 (JP); IRIE, Yukio, Gunma 379-21 (JP)
(74) Representative: Türk, Gille, Hrabal, Leifert
(86) International application number: JP9501670
(87) International publication number: WO9607414

(57) **Abstract**

An ointment which contains 9-[(1R, 2R, 3S)-2,3-bis(hydroxymethyl)cyclobutan-1-yl]guanine and an oleaginous base as the essential ingredient and wherein the guanine powder is homogeneously dispersed in the ointment base. An example thereof comprises 0.05 - 10 % of the guanine, 40 - 99.5 % of an oleaginous base, and if necessary a surfactant as an agent for imparting skin affinity and water as a base. The ointment is useful as an antiviral agent for topical application and exhibits an excellent therapeutic effect.

## Description

### Technical Field

The present invention relates to an ointment preparation containing 9-[(1R, 2R, 3S)-2,3-bis(hydroxymethyl)cyclobutan-1-yl]guanine (hereinafter referred to as compound A) which is useful as antiviral agent.

### Background Art

The compound A is a known compound obtained by synthesis, which is one of the compounds of cyclobutane derivatives, and the usefulnesses thereof for antiviral agent and antitumor agent are expected. There are disclosed in the prior art references in that the cyclobutane derivatives can be used as in dosage forms such as ointment, cream, aerosol, gel preparation, powder, lotion, suspention or solution for local administration [JP-A-2-6478 (1990) and 3-95165 (1991)].

In therapy of viral infectious diseases, particularly in case of curing infectious diseases of the external tissues, such as the skin, mouth, eyes and the like, an antiviral agent may preferably be applied to the diseased portion in the patient by local administration, rather than by systemic administration. However, in case of making a pharmaceutical preparation containing the compound A for local administration, a pharmaceutical preparation in suspension state can only be obtained, for the reason that the compound A is slightly soluble in water and is quite hardly soluble in other solvents. Generally, the manifestation of therapeutic effect performed by a pharmaceutical preparation in suspension state may be varied easily depending on the additives being used therein, so that a pharmaceutical preparation containing the compound A having a high therapeutic effects is earnestly desired.

### Disclosure of Invention

As the result of an extensive research work conducted by the present inventors, they found the fact that a pharmaceutical preparation of ointment containing an oleaginous base, in which powder of the compound A are dispersed uniformly, performs the higher therapeutic effect as compared with that of shown by an ointment preparation containing no oleaginous base, and finally the present invention was completed.

Thus, the present invention relates to the following ointment prepararion.
(1) An ointment preparation characterized by containing the compound A and an oleaginous base as the essential ingredients, and powder of the compound A are dispersed uniformly in the oleaginous base.
And more specifically as follows.
(2) The ointment preparation according to the above-mentioned (1), wherein the content of the compound A in the ointment preparation is 0.05 ∼ 10%.
(3) The ointment preparation according to the above-mentioned (1), wherein the content of the compound A in the ointment preparation is 0.5 ∼ 3%.
(4) The ointment preparation according to the above-mentioned (1), (2) or (3), wherein the oleaginous base is a gelated hydrocarbon.
(5) The ointment preparation according to the above-mentioned (1), (2) or (3), which contains a surface active agent, wherein the content of the surface active agent in the ointment preparation is 50% or less.
(6) The ointment preparation according to the above-mentioned (5), which contains water, wherein the content of water in the ointment preparation is 50% or less.
(7) An ointment preparation containing the compound A, an oleaginous base, and if necessary a surface active agent and water, and the contents of these ingredients in the whole body of ointment preparation are as follows:

| | |
|---|---|
| The compound A | 0.05 ∼ 10% |
| An oleaginous base | 99.95 ∼ 40% |
| A surface active agent | 0 ∼ 50% |
| Water | 0 ∼ 50%. |

(8) The ointment preparation according to the above-mentioned (7), wherein the contents of each one of these ingredient in the whole body of ointment preparation are as follows:

| | |
|---|---|
| The compound A | 0.5 ∼ 3% |
| An oleaginous base | 99.5 ∼ 50% |
| A surface active agent | 0 ∼ 30% |
| Water | 0 ∼ 30%. |

(9) An ointment preparation substantially not containing a surface active agent and water, and containing 0.5 ∼ 3% of the compound A, and 99.5 ∼ 97% of an oleaginous base.

### Brief Description of Drawing

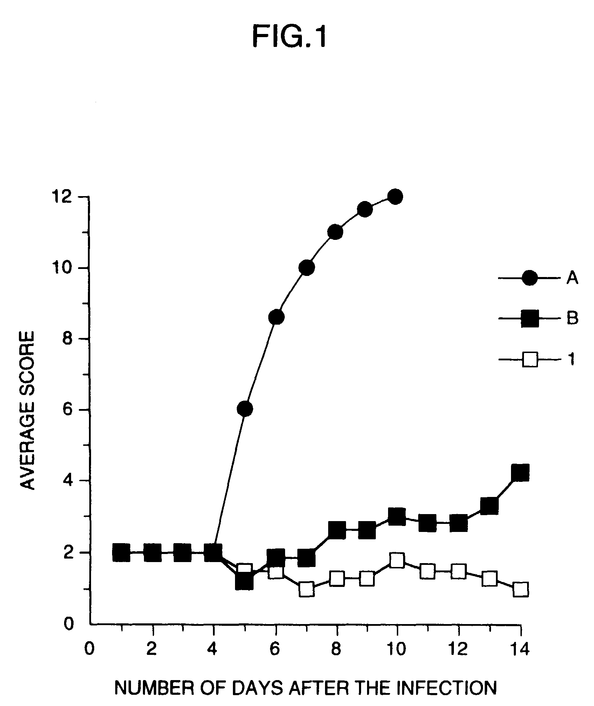

Fig. 1 shows the changes in average scores of lesion of the skin which was induced by inoculation with herpes simplex virus on the dorsal skin of mouse, as in Test Example 1 mentioned later, against the number of days after the infection with herpes simplex virus, wherein the curve 1 indicates the changes in average scores obtained from the test group in which the test mouse was administered by coating with the ointment preparation of the present invention containing 1% of the compound A; the curve A indicates the changes in average scores obtained from the negative control: sham treatment of control group; and the curve B indicates the changes in average scores obtained from the positive control group in which the test mouse was administered by coating with a macrogol ointment containig 1% of the compound A.

### Best Mode for Carrying out the invention

The present invention will be explained in detail as follows. In the present specification, unless otherwise provided, the "%" means % by weight. The compound A may be used either in crystalline state or amorphous state.

The content of the compound A in the ointment preparation is generally 0.05% or more, preferably 0.1% or more, more preferably 0.3% or more, and specifically preferably 0.5% or more, and the upper limit thereof is generally 10%, preferably 5%, more preferably 4%, and specifically prefereably 3%. Therefore, the content of the compound A in the ointment preparation is generally 0.05 ∼ 10%, preferably 0.1 ∼ 5%, more preferably 0.3 ∼ 4%, and specifically preferably 0.5 ∼ 3%.

As to the oleaginous bases, there can be exemplified hydrocarbons, fats and fatty oils, waxes, silicone oils and the like, and among these, hydrocarbons are preferable.

As to the hydrocarbons, there can be exemplified gelated hydrocarbons (liquid paraffin containing polyethylene having a molecular weight of 3500 or more, preferably liquid paraffin containing 2 ∼ 8% of polyethylene having a molecular weight of 3500 ∼ 35000), heavy grade liquid paraffin, light grade liquid paraffin, white petrolatum, yellow petrolatum, squalene and the like. As to the fats and fatty oils, medium chain fatty acid triglycerides, synthetic oils such as hardened fat, vegetable oils such as olive oil, soybean oil, peanut oil, safflower oil, rice oil, sesame oil, camelia oil, corn oil, cotton seed oil, coconut oil and the like, animal oils and fats such as lard, beef tallow and the like and hardened oils of these can be exemplified. As to the waxes, natural waxes such as yellow bees wax, calnauba wax and the like, petroleum waxes such as paraffin, micro-crystalline wax and the like, mineral waxes such as montan wax and the like, and synthetic wax can be exemplified. Furthermore, mixtures of these oleaginous bases can be used. Among these, the gelated hydrocarbons can be exemplified as preferable oleaginous base.

In order to reinforce the affinity of the ointment to the skin, the ointment preparation of the present invention may contain a surface active agent. As to the surface active agent, there can be exemplied a nonionic surface active agent and/or an anionic surface active agent. As to the nonionic surface active agents, there can be exemplified polyoxyethylene-polyoxypropylene glycols, aliphatic alcohols, fatty acid esters of glycerin, fatty acid esters of sorbitan, fatty acid esters of polyoxyethylene sorbitan and the like. As to the anionic surface active agents, there can be exemplified fatty acids, sulfate esters, polyacrylic acids, carboxyvinyl polymers, cellulose derivatives and their alkali metal salts and the like. Among these, the nonionic surface active agents are preferable. In case of using the surface active agents, the content thereof in the ointment preparation is generally 50% or less, preferably 30% or less, for example generally 2 ∼ 50%, preferably 5 ∼ 30%.

The ointment preparation may be in the form of emulsion which further contains water in addition to surface active agents. As to the form of emulsion, either W/O type emulsion or O/W type emulsion may be used, and an ointment preparation of W/O type emulsion which is able to contact easily to the skin is preferable. In case of using water, the content thereof in the ointment preparation is generally 50% or less, preferably 30% or less, for example generally 5 ∼ 50%, preferably 10 ∼ 30%.

Other additives, for example antioxidants such as phenols and the like, or preservatives such as benzoates and the like may be added to the ointment preparation of the present invention. In case of adding these additives, the content thereof in the ointment preparation is generally 0.01 ∼ 1%.

The ointment preparation of the present invention may be directly applied to the diseased portion by coating, or may be reformed in a patch sheet preparation such as a cataplasma and plaster in which said ointment preparation is coated thereon.

In the ointment preparation of the present invention, the amounts of the compound A, oleaginous base, and optional ingredients, such as surface active agent and water to be contained in the whole body of the ointment preparation may be preferably as follows.
- The compound A:: 0.05% or more, preferably 0.1% or more, more preferably 0.3% or more, and specifically preferably 0.5% or more, the upper limit thereof is generally 10%, preferably 5%, more preferably 4% and specifically preferably 3%, and, therefore, generally 0.05 ∼ 10%, preferably 0.1 ∼ 5%, more preferably 0.3 ∼ 4%, and specifically preferably 0.5 ∼ 3%.
- Oleaginous base:: 99.95 ∼ 40%, preferably 99.5 ∼ 50%.
- Surface active agent:: May not be added. In case of using, 50% or less, preferably 30% or less, for example 0 ∼ 50%, preferably 0 ∼ 30% may be added.
- Water:: May not be added. In case of using, 50% or less, preferably 30% or less, for example 0 ∼ 50%, preferably 0 ∼ 30% may be added.
- Antioxidant and Preservative:: May not be added. In case of using, 0.01 ∼ 1% may be added.

Further, in order to expect the localized effect, the ointment preparation may have preferably the characteristics of the lesser translocation of the compound A into the blood, and of the larger effect for inhibiting the extension of the skin lesion, as to such examples, ointment preparations substantially not containing a surface active agent and water can be exemplified.

The oleaginous ointment preparation of the present invention is prepared by a common method. Thus, the compound A is dispersed uniformly by kneading with the above-mentioned oleaginous base, if necessary, further with a surface active agent, water as well as other additives, and when the mixture is in high viscosity, the mixture may be heated. In case of using water as an optional ingredient, water is added to the mixture at the last step of kneading. The compound A may be ground previously, or may be ground during the kneading-dispersion step. In case where the particles of the compound A are large in size, then it is preferable to ground the particles with a liquid hydrocarbon. Particle size of the compound A being dispered in the ointment preparation is generally less than 60 µm as an average particle size regarded as circular diameter, preferably less than 30 µm, which is measured under a microscopic observation, and at least 80% of the number of the particles are preferably having the particle size within the range of 1 ∼ 100 µm, more preferably within the range of 2 ∼ 50 µm.

Next, the action and effect of the present invention will be shown specifically by Test Examples as follows.

### Test Example 1. In vivo test of anti-herpes viruses effects

### (1) Samples for the test

- Present invention:: Ointment preparation containing 1% of the compound A of the Example 1
- Positive control group:: Macrogol ointment preparation containing 1% of the compound A of the Reference Example 1
- Negative control:: Sham treatment

### (2) Test method

The dorsum hair of test mouse was removed by chemical depilatory, and the depilated skin of 5 × 5 mm² in size was scratched 10 times by using a needle of 26G for intracutaneous injection. A liquid containing 2.8 × 10⁵ particles/20 µl of herpes simplex virus (HSV-2, Strain 186) were inoculated by dropping to the scratched portion of the skin of test mouse. 3 Hours after the inoculation with the virus, about 20 mg of the ointment preparation of the present invention or the macrogol ointment preparation was coated, respectively on the inoculated portion of each one of the test mouse once in the first day, and then twice a day for five consecutive days. Test groups for the administration and control group were consisting of 10 mice, and the extensions of the skin lesions during 14 days after the inoculation with the virus were observed in every day, and the results were evaluated by converting the extension of the skin lesions into scores as shown in Table 1.

**Table 1**

| Skin lesion score | |
|---|---|
| Score | State of the skin lesion |
| 0 | No lesion |
| 2 | Localized skin lesion |
| 6 | Slight zosteriform skin lesion |
| 8 | Moderate zosteriform skin lesion |
| 10 | Severe zosteriform skin lesion |
| 12 | Death |

### (3) Test results

Anti-herpes viruses effects of the ointment preparation of the present invention compared with those of the positive and negative controls with respect to the skin lesions are shown in Fig. 1.

The ointment preparation of the present inven tion containing the compound A remarkably inhibits the extension of the skin lesion, thus anti-herpes viruses effects were acknowledged. As compared with the macrogol ointment preparation containing 1% of the compound A, the ointment preparation of the present invention containing 1% of the compound A can remarkably inhibit the extension of the skin lesion, thus anti-herpes viruses effects performed by the ointment preparation of the present invention were acknowledged. In Fig. 1, the curve 1 shows the data of test group wherein the ointment preparation of the present invention containing 1% of the compound A was administered by coating; the curve A shows the data of sham treatment group; and the curve B shows positive control wherein the macrogol ointment preparation containing 1% of the compound A was administered by coating. These curves of 1, A and B show the changes of average scores of the skin lesions against the number of days after the infection.

### Test Example 2. In vivo test of anti-herpes viruses effects

Test and evaluation were conducted by methods identical to those employed in Test Example 1, execept that herpes simplex virus HSV-1 (Strain KOS) was used, in place of the herpes simplex virus HSV-2 (Strain 186), that a liquid containing 3.8 × 10⁷ particles/20 µl of HSV-1 (Strain KOS) was inoculated by dropping to test mouse and that 3 hours after the inoculation with virus, the ointment preparation of the present invention (the ointment preparation of Example 1, containing 1% of the compound A or the ointment preparation of Example 7, containing 3% of the compound A) was coated, respectively on the anticipated site of lesion to be manifested (the right abdomen region of test mouse) in an amount of about 30 mg per one mouse, respectively once in the first day, and then twice a day for seven consecutive days. The results are shown in Table 2.

**Table 2**

| Average scores | | | |
|---|---|---|---|
| Number of days after the inoculation | Control group (No treatment was made) | Test groups coated with the ointment preparation of the present invention (Content of the compound A) | |
| | | 1% | 3% |
| 1 | 2 | 2 | 2 |
| 2 | 2 | 2 | 2 |
| 3 | 2 | 2 | 2 |
| 4 | 2 | 2 | 2 |
| 5 | 4.4 | 2 | 2 |
| 6 | 6.4 | 2.2 | 1.2 |
| 7 | 7.4 | 1.8 | 1 |
| 8 | 7.6 | 1.4 | 1 |
| 9 | 7.2 | 1.2 | 0.2 |
| 10 | 6.2 | 0.8 | 0.2 |
| 11 | 4.8 | 0.8 | 0 |
| 12 | 4.4 | 0.8 | 0 |
| 13 | 4.4 | 0.6 | 0 |
| 14 | 2.8 | 0.6 | 0 |

It can be seen obviously from Table 2 that the ointment preparation of the present invention containing the compound A remarkably inhibits the extension of the skin lesion.

As explained above, the oinment preparation of the present invention can be used as antiviral agents. Especially, it can be used for local therapy of herpes simplex, such as herpes febrilis, herpes facialis, herpes genitalis, herpes gluteal region, Kaposi varicelliform eruption, hepatic felon and other cutaneous herpes simplexes which are induced by herpes simplex virus type 1 (HSV-1) and herpes simplex virus type 2 (HSV-2), as well as varicella, and zoster herpes which are induced by varicella zoster virus (VZV).

Next, some of the ointment preparations of the present invention are shown by the following examples.

### Example 1

0.3 Grams of the compound A and 0.3 g of liquid paraffin (Miyazawa Yakuhin Co.) were taken in a mortar and were ground. To this mixture was added 29.4 g of gelated hydrocarbon ("Plastibase", Bristol-Myers Squibb Co.) and the whole mixture was kneaded and dispersed uniformly. This ointment was filled in an aluminum tube container and made it as 1% ointment preparation of the compound A. The particle size of the compound A in this ointment preparation was 13 µm as an average particle diameter regarded as a circle, measured by use of an image analyzing apparatus (XL 500, Olympus Optical Co., Ltd) with a microscopic observation, and 80% of the number of these particles had the particle size within the range of 3 ∼ 16 µm.

### Example 2

0.09 Grams of the compound A and 0.3 g of liquid paraffin were taken in a mortar and were ground. To this mixture was added 29.61 g of gelated hydrocarbon and the whole mixture was kneaded and dispersed uniformly. This ointment was filled in an aluminum tube container to make it as 0.3% ointment preparation of the compound A.

### Example 3

0.03 Grams of the compound A and 0.3 g of liquid paraffin were taken in a mortar and were ground. To this mixture was added 29.67 g of gelated hydrocarbon and the whole mixture was kneaded and dispersed uniformely. This ointment was filled in an aluminum tube container to make it as 0.1% ointment preparation of the compound A.

### Example 4

0.3 Grams of the compound A and 0.3 g of liquid paraffin were taken in a mortar and were ground. To this mixture was added 26.4 g of gelated hydrocarbon and 3 g of polyoxyethylene[160]polyoxypropylene[30] glycol (Pluronic F68, Asahi Denka Kogyo K. K.), then the whole mixture was heated to 70°C and was kneaded and dispersed uniformely. This ointment was filled in an aluminum tube container to make it as 1% ointment preparation of the compound A.

### Example 5

0.3 Grams of the compound A and 0.3 g of liquid paraffin were taken in a mortar and were ground. To this mixture was added 26.4 g of gelated hydrocarbon and 3 g of polyoxyethylene[160]polyoxypropylene[30] glycol (Pluronic F68, Asahi Denka Kogyo K. K.), then the whole mixture was heated to 70°C and was kneaded and dispersed uniformely. Then 6 g of purified water was added thereto and kneaded. This ointment was filled in an aluminum tube container to make it as 1% ointment preparation of the compound A.

### Example 6

0.3 Grams of the compound A was taken in a mortar and ground. Then 29.7 g of a water absorbing ointment base (containing white petrolatum and cetanol as the main ingredient) (Hoei Yakkou Co.) was added thereto and they were kneaded and dispersed uniformly. This ointment was filled in an aluminum tube container to make it as 1% ointment preparation of the compound A.

### Example 7

0.9 Grams of the compound A and 0.9 g of liquid paraffin (Miyazawa Yakuhin Co.) were ground in a mortar. Then 28.2 g of gelated hydrocarbon (Plastibase) was added thereto and they were kneaded and dispersed uniformly. This ointment was filled in an aluminum tube container to make it as 3% ointment preparation of the compound A.

### Reference Example 1

0.3 Grams of the compound A and 0.3 g of macrogol 400 (Nippon Oil and Fats Co., Ltd.)(water-soluble base) were taken in a mortar and were ground. Then 29.4 g of macrogol ointment base (Yoshida Seiyaku Co.)(water-soluble base) was added thereto and the whole mixture was kneaded and dispersed uniformly. This ointment was filled in an aluminum tube container to make it as 1% ointment preparation of the compound A. The particle size of the compound A in this ointment preparation was 15 µm as an average particle diameter regarded as a circle, measured by use of an image analyzing apparatus (XL 500, Olympus Optical Co., Ltd) with a microscopic observation, and 80% of the number of these particles had the particle size within the range of 3 ∼ 18 µm.

### Industrial Applicability

The oleaginous ointment preparation of the present invention possesses high therapeutic effect as compared with an ointment preparation using other ointment base, and thus useful as antiviral agent.

## Claims

1. An ointment preparation characterized by containing 9-[(1R, 2R, 3S)-2,3-bis(hydroxymethyl)cyclobutan-1-yl]guanine and oleaginous ointment bases as the essential ingredients, and powder of 9-[(1R, 2R, 3S)-2,3-bis(hydroxymethyl)cyclobutan-1-yl]guanine is dispersed uniformly in the ointment base.

2. The ointment preparation according to Claim 1, wherein the content of 9-[(1R, 2R, 3S)-2,3-bis(hydroxymethyl)cyclobutan-1-yl]guanine in the ointment preparation is 0.05 ∼ 10%.

3. The ointment preparation according to Claim 1, wherein the content of 9-[(1R, 2R, 3S)-2,3-bis(hydroxymethyl)cyclobutan-1-yl]guanine in the ointment preparation is 0.5 ∼ 3%.

4. The ointment preparation according to Claim 1, 2 or 3, wherein the oleaginous ointment base is a gelated hydrocarbon.

5. The ointment preparation according to Claim 1, 2 or 3, which contains a surface active agent, and the content of the surface active agent is 50% or less in the ointment preparation.

6. The ointment preparation acording to Claim 5, which contains water, and the content of water is 50% or less in the ointment preparation.

7. An ointment preparation containing 9-[(1R, 2R, 3S)-2,3-bis(hydroxymethyl)cyclobutan-1-yl]guanine (compound A), oleaginous bases, and if necessary containing an surface active agent and water, and the contents of these ingredients in the whole body of the ointment preparation are as follows.
| | |
|---|---|
| Compound A | 0.05 ∼ 10% |
| Oleaginous base | 99.95 ∼ 40% |
| Surface active agent | 0 ∼ 50% |
| Water | 0 ∼ 50% |

8. The ointment preparation acording to Claim 7, wherein the contents of each one of the ingredients in the whole body of the ointment preparation is as follows.
| | |
|---|---|
| Compound A | 0.5 ∼ 3% |
| Oleaginous base | 99.5 ∼ 50% |
| Surface active agent | 0 ∼ 30% |
| Water | 0 ∼ 30% |

9. An ointment preparation substantially not containing a surface active agent and water, and containing 0.5 ∼ 3% of 9-[(1R, 2R, 3S)-2,3-bis(hydroxymethyl)cyclobutan-1-yl]guanine and 99.5 ∼ 97% of oleaginous bases.
